# EUROPÄISCHE PATENTANMELDUNG

(11) **EP 3 517 022 A1**
(43) Veröffentlichungstag der Anmeldung: **31.07.2019**
(21) Anmeldenummer: 19152814.0
(22) Anmeldetag: 21.01.2019
(51) Int. Cl.: A61B 3/135, A61B 3/14

(54) **AUGENUNTERSUCHUNGSGERÄT**

(30) Priorität: 25.01.2018 CH 882018
(71) Anmelder: HAAG-STREIT AG, 3098 Köniz (CH)
(72) Erfinder: Breitenstein, Jörg, 3052 Zollikofen (CH); Zumkehr, Frank, 3052 Zollikofen (CH); Dellagiacoma, Claudio, 3013 Bern (CH)
(74) Vertreter: Keller & Partner Patentanwälte AG

(57) **Zusammenfassung**

Eine Vorrichtung (1) zur Untersuchung eines Auges, insbesondere eine Spaltlampe, umfasst eine Bildaufnahmeeinheit (220) mit mindestens einem ersten Sensor (241.1) in einem ersten Strahlengang und einem zweiten Sensor (241.2) in einem zweiten Strahlengang, wobei die Vorrichtung im ersten Strahlengang weiter ein erstes Objektiv mit einer ersten optischen Achse umfasst, welches eine fixe Vergrösserung aufweist und fix im ersten Strahlengang angeordnet ist.

## Beschreibung

### Technisches Gebiet

Die Erfindung betrifft eine Vorrichtung zur Untersuchung eines Auges, insbesondere eine Spaltlampe, umfassend eine Bildaufnahmeeinheit mit mindestens einem ersten Sensor in einem ersten Strahlengang und einem zweiten Sensor in einem zweiten Strahlengang. Weiter betrifft die Erfindung ein Verfahren zum Ermitteln von Bilddaten mit einer Vorrichtung zur Untersuchung eines Auges.

### Stand der Technik

Spaltlampenmikroskope sind ophthalmologische Untersuchungsgeräte, mit welchen die Augen monoskopisch oder stereoskopisch untersucht werden können. Bekannte Spaltlampenmikroskope haben zur stereoskopischen Betrachtung eines Auges eine Optik zum Erzeugen zweier Bilder eines Auges und zwei Okulare zur stereoskopischen Betrachtung der Bilder sowie eine Beleuchtungseinheit. Die Beleuchtungseinheit ist auf einem vertikal verlaufenden Ast einer Halteeinheit angeordnet. Das zu betrachtende Auge ist in einer annähernd horizontal verlaufenden Ebene auf einer Seite der Halteeinheit positionierbar. Die Beleuchtungseinheit umfasst eine Glühlampe, LEDs oder ähnliche Beleuchtungsmittel für eine Spaltbeleuchtung.

Aus der EP 2 446 812 B1 (Haag-Streit) ist eine Spaltlampe bekannt, welche zwei Bildsensoren zur elektronischen Erfassung zweier Bilder umfasst, um eine dreidimensionale Darstellung zu erreichen, wobei auch mehr als zwei Bildsensoren vorgesehen sein können. Weiter können Bildsensoren mit unterschiedlichen Spektralbereichen vorgesehen sein. Die elektronisch erfassten Bilddaten können während der Darstellung bearbeitet, insbesondere optimiert, abstrahiert etc. werden.

Die US 2010/201799 A (Zeiss) betrifft eine ophthalmologische Vorrichtung zum Erreichen von Bildern mit erweitertem dynamischem Bereich. Die Vorrichtung umfasst mindestens einen Strahlteiler und mindestens zwei Bildsensoren, sowie eine Spaltlampe oder eine Funduskamera. Damit können mehrere Bilder mit identischer Szene aufgenommen und zu einem Gesamtbild zusammengesetzt werden. Der Strahlteiler hat eine asymmetrische Teilungseinstellung.

Die DE102010014114 (Zeiss) betrifft eine Anwendung einer Bildaufnahmeeinheit eines ophthalmologischen Geräts für Justier- und Messaufgaben. Die Bildaufnahmevorrichtung umfasst eine Digitalkamera mit hoher Auflösung und einer Digitalzoomfunktion.

Die bekannten Vorrichtungen zur Untersuchung eines Auges haben den Nachteil, dass diese komplex und teuer in der Herstellung sind.

### Darstellung der Erfindung

Aufgabe der Erfindung ist es, eine dem eingangs genannten technischen Gebiet zugehörende Vorrichtung zur Untersuchung eines Auges zu schaffen, welche besonders kostengünstig und robust ausgebildet ist.

Die Lösung der Aufgabe ist durch die Merkmale des Anspruchs 1 definiert. Gemäss der Erfindung umfasst die Vorrichtung im ersten Strahlengang weiter ein erstes Objektiv mit einer ersten optischen Achse, welches eine fixe Vergrösserung aufweist und fix im ersten Strahlengang angeordnet ist.

Optomechanische Teile sind in der Herstellung sehr teuer, da diese äusserst präzise und robust sein müssen. Dadurch dass das Objektiv eine fixe Vergrösserung aufweist, kann die Anzahl bewegter Teile in der Vorrichtung reduziert werden, womit die Vorrichtung als Ganzes kostengünstiger produziert werden kann.

Nachfolgend wird unter dem Begriff "a und/oder b" jeweils eine nichtleere Teilmenge der Elemente a und b verstanden, das heisst entweder a oder b oder (a und b).

Unter dem Begriff "fix im Strahlengang angeordnet" wird vorliegend verstanden, dass das Objektiv im Strahlengang verbleibt - dem Fachmann ist allerdings klar, dass das Objektiv den Strahlengang (mit) definiert. Mit der fixen Anordnung im Strahlengang ist damit zu verstehen, dass die Vorrichtung nicht derart ausgebildet ist, dass das Objektiv im Strahlengang nicht durch ein anderes Objektiv ersetzbar ist. Eines oder mehrere der Objektive können jedoch verschwenkbar oder verschiebbar ausgebildet sein, wobei durch entsprechende Verschwenkung oder Verschiebung des Objektivs auch der Strahlengang geändert wird.

Die **Vorrichtung** ist vorzugsweise als Spaltlampe ausgebildet, womit in einer bevorzugten Anwendung ein vorderer Augenabschnitt untersucht werden kann. Dem Fachmann ist aber klar, dass die Erfindung auch auf verwandte Vorrichtungen übertragbar ist, insbesondere kann die Erfindung auch bei einer Funduskamera oder dergleichen realisiert werden. Unter dem Begriff "Spaltlampe" ist eine Vorrichtung zur stereoskopischen Untersuchung eines Auges zu verstehen, welche eine Vorrichtung zur Spaltbeleuchtung umfasst. Dem Fachmann sind solche Geräte hinreichend bekannt.

Die **Bildaufnahmeeinheit** umfasst mindestens einen ersten und einen zweiten Sensor. Die Sensoren sind damit vorzugsweise zum Aufnehmen eines Bildes geeignet. Der Sensor muss dabei nicht zwingend im sichtbaren Spektralbereich arbeiten (siehe unten). Der Sensor kann unter Umständen aus mehreren einzelnen Sensoren zusammengesetzt sein. Nachfolgend wird unter Sensor jeweils ein einem Objektiv zugeordneter Einzelsensor oder ein aus mehreren einzelnen Sensoren bestehender Sensor, welcher ebenfalls einem Objektiv zugeordnet ist, verstanden. Besonders bevorzugt handelt es sich bei dem ersten und/oder dem zweiten Sensor um einen digitalen Sensor. Insbesondere da in der bevorzugten Ausführungsform, neben allfällig mehreren vorhandenen Objektiven mit unterschiedlicher fixer Vergrösserung, keine optomechanische Vergrösserung vorgesehen ist, können mit den digitalen Sensoren besonders einfach, insbesondere unter Verwendung bekannter Techniken (Interpolation etc.), digitale Bildvergrösserungen vorgenommen werden. Dadurch, dass ein erster Sensor und ein zweiter Sensor vorgesehen sind, ist bei geeigneter Ausrichtung der beiden Sensoren eine stereoskopische Aufnahme möglich. Zudem können Bilddaten mehrerer Sensoren desselben Objektausschnitts zu einem einzigen Bild verrechnet werden. Damit kann die Bildqualität gegenüber den Einzelbildern erheblich verbessert werden. Damit kann mit mehreren Einzelsensoren respektive mit mehreren Objektiven ein Sensor simuliert werden, welcher eine höhere Auflösung aufweist, als die einzelnen Sensoren. Damit kann eine in Relation zur erreichbaren Auflösung relativ kostengünstige Vorrichtung erreicht werden.

Im ersten **Strahlengang** befindet sich der erste Sensor und in einem zweiten Strahlengang befindet sich der zweite Sensor. Der Begriff "Strahlengang" wird durch diejenigen Lichtstrahlen definiert, welche ausgehend vom beobachteten Objekt den Sensor erreichen. Die erfindungsgemässe Vorrichtung kann zusätzlich zu dem Sensor einen Strahlteiler für ein Okular umfassen. Die Lichtstrahlen, welche in diesem Fall vom Objekt zum Okular gelangen, werden, sofern nichts anderes gesagt ist, nicht dem Strahlengang zugeordnet. In der bevorzugten Ausführungsform umfasst die Vorrichtung jedoch keine Okulare im klassischen Sinne, sondern allenfalls elektronische, mit Bildschirmen versehende Okulare oder dergleichen. Der Strahlengang ist typischerweise ein polygonaler Streckenzug, welcher ein Lichtstrahl ausgehend vom Objekt durch die optischen Elemente (Linsen, Spiegel etc.) bis hin zum Sensor zurücklegt. Je nach Ausbildung der optischen Elemente kann der Strahlengang aber auch als Gerade ausgebildet sein oder Krümmungen aufweisen.

Unter dem Begriff **Objektiv** wird nachfolgend das optische Element der Vorrichtung verstanden, welches im Strahlengang adjazent zum beobachteten optische Element angeordnet ist. Das heisst, das Objektiv liegt zwischen dem Bild und dem Objekt.

Erfindungsgemäss weist das erste Objektiv eine **fixe Vergrösserung** auf. Damit weist das erste Objektiv eine fixe Brennweite auf. Objektive mit fixer Brennweite ermöglichen typischerweise Aufnahmen mit besserer Bildqualität gegenüber Zoomobjektiven, da diese einen einfacheren Aufbau mit weniger respektive weniger bewegbaren Elementen aufweisen.

Prinzipiell wäre es denkbar, das erste Objektiv mit der fixen Brennweite auswechselbar vorzusehen, so dass während der Aufnahmen unterschiedliche Brennweiten durch unterschiedliche Objektive erreicht werden könnten. Erfindungsgemäss wird aber auf diese Auswechselbarkeit verzichtet, so dass das erste Objektiv mit der fixen Vergrösserung im ersten Strahlengang fix angeordnet ist. Bewegbare Teile stellen in der Regel eine Fehlerquelle für die Vorrichtung dar. Insbesondere wenn sich die bewegbaren Teile als optische Elemente im Strahlengang befinden oder in diesen einsetzbar sind, können bereits minimale Abweichungen von der Sollposition zu Abbildungsfehlern führen. Dadurch, dass das erste Objektiv fix im ersten Strahlengang angeordnet ist, wird eine besonders robuste und präzise Vorrichtung erreicht. Dadurch, dass auf aufwändige optomechanische Bauteile verzichtet werden kann, wird die Vorrichtung zudem als Ganzes kostengünstiger.

Besonders bevorzugt ist die Vorrichtung ohne Okular für eine analoge Betrachtung des Objekts ausgebildet. Die Vorrichtung ist damit bevorzugt ausschliesslich zur digitalen Darstellung der durch die Bildsensoren erfassten Bilddaten ausgelegt. Dadurch, dass die Bilddaten digitalisiert sind, kann zur Betrachtung der Bilddaten eine Darstellungseinheit, insbesondere ein elektronisches Display oder ähnliches, verwendet werden, welches unabhängig von der Optik, insbesondere unabhängig von den Okularen positionierbar ausgebildet sein kann. Damit kann für den Anwender eine besonders ergonomische Haltung während der Anwendung erreicht werden. Dazu kann die Vorrichtung einen oder mehrere elektronische Bildschirme respektive Displays aufweisen. Die Mittel zur Darstellung der Bilddaten müssen nicht zwingend durch die Vorrichtung selbst umfasst sein. Die Vorrichtung kann auch lediglich dazu ausgelegt sein, die Bilddaten zu erfassen, während die Darstellung der Bilddaten über ein separates Gerät erfolgt. Damit kann die Vorrichtung als Ganzes weiter vereinfacht werden, insbesondere da die Okulare und deren Linsensysteme teuer in der Herstellung sind.

In Varianten kann die Vorrichtung, zum Beispiel über einen Strahlteiler, in einem oder in beiden Strahlengängen ein Okular umfassen, womit das Objekt in klassischer Weise analog (d.h. nicht digitalisiert) betrachtet werden kann.

Vorzugsweise umfasst die Vorrichtung eine Fokusebenenumschaltung, womit zwischen zwei oder mehr Fokusebenen umgeschaltet werden kann. Bevorzugt umfasst die Vorrichtung für jeden Sensor eine solche Fokusebenenumschaltung.

In Varianten kann auch eine stetige Einstellung der Fokusebene vorgesehen sein. Alternativ kann auf die Fokusebenenumschaltung auch verzichtet werden. In diesem Fall kann zum Beispiel die Vorrichtung relativ zum Objekt derart bewegbar sein, dass die Fokalebene eingestellt werden kann. Dazu kann entweder das Objekt (z.B. eine Kinn- und Stirnstütze bei einer Spaltlampe) oder die Vorrichtung bewegbar sein.

Vorzugsweise umfasst das erste Objektiv den ersten Sensor. Damit wird ein besonders kompakter Aufbau der Vorrichtung erreicht. Insbesondere ist damit in einfacher Weise eine Ausrichtbarkeit des Objektivs auf ein Objekt realisierbar. Weiter umfasst die Vorrichtung vorzugsweise ein zweites Objektiv mit einer zweiten optischen Achse, wobei das zweite Objektiv den zweiten Sensor umfasst sowie insbesondere eine fixe Vergrösserung aufweist und fix im zweiten Strahlengang angeordnet ist. Damit wird gesamthaft ein kostengünstiger Aufbau einer Vorrichtung erreicht, mit welcher ein Auge stereoskopisch betrachtet werden kann. Mit den beiden Objektiven müssen nicht zwingend stereoskopische Aufnahmen erreichbar sein. Die beiden Objektive mit den Sensoren können auch eingesetzt werden, um andere Effekte zu erreichen, insbesondere können mit den beiden Sensoren zum Beispiel unterschiedliche Bereiche des Auges erfasst oder unterschiedliche Spektren abgedeckt werden (siehe unten).

In Varianten kann der Sensor auch im Strahlengang hinter dem Objektiv angeordnet sein, so dass zum Beispiel zwischen dem Objektiv und dem Sensor weitere optische Elemente angeordnet werden können.

Vorzugsweise unterscheiden sich der erste Sensor und der zweite Sensor in einem Aufnahmespektrum. Damit können grössere Spektralbereiche mit grosser Empfindlichkeit abgedeckt werden.

In Varianten können der erste Sensor und der zweite Sensor auch dasselbe Aufnahmespektrum aufweisen. In diesem Fall kann mit den überlappenden Bilddaten der beiden Sensoren ein einziges Bild mit vergrösserter Auflösung und/oder Schärfe erreicht werden. Die beiden Sensoren können insbesondere auch dasselbe Aufnahmespektrum mit jedoch unterschiedlicher Auflösung aufweisen.

Bevorzugt ist der erste Sensor als Farbsensor, insbesondere zum Beispiel als RGBrespektive Weisslicht-Sensor ausgebildet und der zweite Sensor als Schwarzweiss-Sensor oder als IR-Sensor ausgebildet. Der Schwarzweiss-Sensor hat gegenüber dem Farbsensor den Vorteil, dass bei vergleichbarer Pixelzahl höhere Auflösungen möglich sind. Aufgrund der Farbfilter bei den Farbsensoren, insbesondere des Bayer-Filters, wird die Auflösung in der Regel um ungefähr einen Faktor 2 reduziert. Anderseits hat der Farbsensor den Vorteil, dass die Farbaspekte, welche bei der Augenuntersuchung sehr hilfreich sein können, erfasst werden können. Durch die Kombination eines Farbsensors als ersten Sensor mit einem Schwarzweiss-Sensor als zweiten Sensor können nun die Vorteile der beiden Sensoren kombiniert werden.

Bevorzugt werden die Bilddaten eines als Schwarzweiss-Sensor ausgebildeten ersten Sensors mit einer ersten Auflösung, mit Bilddaten eines als Farb-Sensor ausgebildeten zweiten Sensors zu einem einzigen kolorierten Bild und/oder zu einer kolorierten Bildsequenz verrechnet, wobei der zweite Sensor eine Auflösung aufweist, welche insbesondere geringer ist als die Auflösung des ersten Sensors. Dadurch, dass der zweite Sensor, das heisst der Farbsensor, eine geringere Auflösung aufweist, als der Schwarzweiss-Sensor, kann ein besonders kostengünstiges Verfahren geschaffen werden, um hochauflösende, aber dennoch farbige Bilddaten zu erhalten.

In der Kombination eines im sichtbaren Bereich operierenden Sensors mit einem IR-Sensor können weiter zum Beispiel Entzündungen im Auge festgestellt werden, da entzündete Stellen in der Regel eine erhöhte Temperatur aufweisen.

Schliesslich können auch Sensoren mit anderen Spektralbereichen eingesetzt werden, insbesondere Sensoren mit engen Spektralbereichen, welche so auch in der chemischen Analytik eingesetzt werden.

Alternativ kann einer der Sensoren auch als UV-Sensor ausgebildet sein. Damit könnte ein UV-Sensitiver Marker im Objekt beobachtet werden. Dem Fachmann sind weitere Sensortypen und Anwendungen zu den Sensortypen bekannt.

Vorzugsweise weist die Vorrichtung mindestens einen dritten Sensor in einem dritten Strahlengang auf. Mit dem dritten Sensor kann das Anwendungsgebiet der Vorrichtung weiter erweitert werden. Der dritte Sensor kann zum Beispiel zur Erstellung eines Übersichtbildes vorgesehen sein, während der erste und der zweite Sensor einen Bildausschnitt des Übersichtsbildes erfassen können.

Auch der dritte Sensor kann sich von einem oder beiden anderen Sensoren in einem Aufnahmespektrum unterscheiden. In einer besonders bevorzugten Ausführungsform sind der zweite Sensor als Farbsensor und der dritte Sensor als IR-Sensor ausgebildet, während insbesondere der erste Sensor zum Beispiel als Schwarzweiss-Sensor ausgebildet ist. In dieser Variante können die Vorteile der drei Sensortypen kombiniert werden. Alternativ können auch alle Sensoren dasselbe Aufnahmespektrum aufweisen. Zum Beispiel können alle Sensoren als Farbsensoren oder als Schwarzweiss-Sensoren ausgebildet sein.

Der dritte Sensor ist bevorzugt durch ein fix im dritten Strahlengang angeordnetes drittes Objektiv umfasst und weist vorzugsweise eine fixe Vergrösserung auf. Damit wird wiederum eine besonders robuste und kostengünstige Vorrichtung erreicht. Sofern der dritte Sensor zur Erstellung eines Übersichtbildes ausgebildet ist, kann das dritte Objektiv eine kleinere Vergrösserung aufweisen, als das erste und das zweite Objektiv. Das erste und das zweite Objektiv können in diesem Fall zum Beispiel einen Farbsensor umfassen und dieselbe Vergrösserung aufweisen, so dass diese in herkömmlicher Weise zur stereoskopischen Betrachtung des Objekts, insbesondere eines Auges eingesetzt werden können.

In Varianten kann auf den dritten Sensor auch verzichtet werden. Weiter können auch mehr als drei Sensoren eingesetzt werden.

Vorzugsweise ist ein erster Vergrösserungsfaktor des ersten Objektivs kleiner, als ein zweiter Vergrösserungsfaktor des zweiten Objektivs. Damit kann mit dem ersten Sensor ein grösserer Bildaufnahmebereich abgedeckt werden, als durch den zweiten Sensor. In dieser Ausführungsform kann zum Beispiel mit dem ersten Sensor ein Übersichtsbild geschaffen werden, während mit dem zweiten Sensor ein Ausschnitt des Übersichtbilds in grösserer Auflösung aufgenommen werden kann. Im Verfahren kann ein mit dem ersten Sensor erfasster Bildbereich gezoomt werden, bis die Auflösung des Bildausschnitts des Bildbereichs nicht mehr hinreichend ist. Ab diesem Zeitpunkt kann vom ersten Sensor zum zweiten Sensor gewechselt werden, um bei stärkerer Vergrösserung den Bildausschnitt hochaufgelöst betrachten zu können. Damit können mit kostengünstigen Sensoren dennoch Bildausschnitte mit hoher Auflösung erreicht werden.

In Varianten können das erste Objektiv und das zweite Objektiv auch dieselbe Vergrösserung aufweisen. Insbesondere wenn in allen Strahlengängen auf ein Objektiv mit grosser Vergrösserung verzichtet wird, kann dennoch eine hohe Auflösung durch den Einsatz entsprechend hochauflösender (und damit typischerweise teurerer) Sensoren erreicht werden.

Vorzugsweise überlappt sich ein Bildaufnahmebereich in einer Fokalebene des ersten Strahlengangs mit einem Bildaufnahmebereich in einer Fokalebene des zweiten Strahlengangs zumindest teilweise. Unter dem Begriff Bildaufnahmebereich wird derjenige Bereich in der Fokalebene verstanden, welcher durch die Bildwinkel des Objektivs gegeben sind, d.h. es handelt sich um den durch das Objektiv abbildbaren Bereich. Durch die teilweise Überlappung kann mit den mehreren Sensoren ein grösserer Bildaufnahmebereich abgedeckt werden. Durch die Überlappung des Bildaufnahmebereichs wird zudem erreicht, dass im Überlappungsbereich die Bilddaten doppelt verfügbar sind, so dass zwischen den Einzelaufnahmen ein Übergang optimal berechnet werden kann. Damit können Reflexe und dergleichen anhand der redundanten Informationen digital eliminiert werden.

Es können in einer Ausführungsform zum Beispiel vier Objektive mit jeweils einem Bildsensor vorgesehen sein, welche einen rechteckigen Bereich derart abdecken, dass die Überlappung eine Kreuzform bildet.

In Varianten können die Sensoren auch derart ausgebildet und ausgerichtet sein, dass jeweils unterschiedliche Bildaufnahmebereiche abgedeckt werden. So können zum Beispiel vier Objektive mit jeweils einem Bildsensor derart ausgerichtet sein, dass sich ein Bildaufnahmebereich ohne Überlappung ergibt.

Vorzugsweise liegt der Bildbereich der Fokalebene des zweiten Strahlengangs innerhalb des Bildbereichs der Fokalebene des ersten Strahlengangs. Damit kann mit dem ersten Sensor ein Übersichtsbild und mit dem zweiten Sensor ein Ausschnitt des Übersichtsbildes erfasst werden. Die technische Umsetzung kann auf unterschiedliche Weisen erreicht werden. In einer ersten Variante können der erste und der zweite Sensor unterschiedliche Grössen aufweisen. In einer zweiten Variante können jeweils derselbe Sensor in ein Objektiv mit unterschiedlicher Vergrösserung eingesetzt werden. Weiter können dieselben Objektive mit denselben Sensoren in unterschiedlichen Abständen zum Objekt angeordnet sein. Dem Fachmann sind weitere Variationen bekannt, insbesondere können die obigen Merkmale auch kombiniert werden, so dass zum Beispiel unterschiedliche Objektive in unterschiedlichen Abständen zum Objekt angeordnet sind.

In Varianten können die Fokalebenen des ersten Strahlengangs mit der Fokalebene des zweiten Strahlengangs auch übereinstimmen.

Vorzugsweise ist ein Winkel zwischen der ersten optischen Achse und der zweiten optischen Achse einstellbar. Vorzugsweise sind die optischen Achsen räumlich verstellbar. Dazu kann ein Objektiv zum Beispiel über zwei zueinander rechtwinklige Schwenkachsen verfügen. Damit können die Fokalebenen des ersten Objektivs und des zweiten Objektivs relativ zueinander bewegt werden. Insbesondere bei einem Einsatz des ersten Objektivs zum Erstellen eines Übersichtbildes hat dies den Vorteil, dass zum Beispiel mit dem zweiten Objektiv ein Bildausschnitt gewählt und variiert werden kann. Besonders bevorzugt ist mindestens ein Objektiv mit einer allfälligen grösseren Vergrösserung bewegbar ausgebildet, während ein Objektiv zur Erstellung eines Übersichtbildes nicht zwingend bewegbar ausgebildet sein muss, insbesondere wenn das Objektiv zur Erstellung des Übersichtbildes in der fixen Positionierung den Bildaufnahmebereich vollständig abdeckt. Alternativ können auch mehrere Objektive bewegbar ausgebildet sein.

In Varianten kann ein Winkel zwischen den optischen Achsen des ersten und des zweiten Objektivs auch fix sein.

Vorzugsweise sind die erste optische Achse und die zweite optische Achse parallel einstellbar. Damit kann mit dem ersten Sensor und mit dem zweiten Sensor ein besonders grosser Bildaufnahmebereich abgedeckt werden. Vorzugsweise ist die dritte optische Achse auch zu einer oder beiden anderen optischen Achsen parallel ausrichtbar.

Alternativ kann auf die parallele Ausrichtung der ersten und der zweiten optischen Achse auch verzichtet werden.

Vorzugsweise sind in der ersten optischen Achse und in der zweiten optischen Achse eine Blende und/oder ein optischer Filter angeordnet, welcher insbesondere motorisch betätigbar ist. Die Blende ist vorzugsweise als verstellbare Blende ausgebildet, womit eine vergrösserungsunabhängige Steuerung der Schärfentiefe erreichbar ist. Alternativ kann auf die Verstellbarkeit der Blende auch verzichtet werden. Bei den Filter handelt es sich vorzugsweise um einen oder mehrere wählbare Farbfilter. Bevorzugt ist der Farbfilter motorisch betätigbar, das heisst in einen oder mehrere der Strahlengänge ein- und ausfahrbar. Damit kann die Handhabung der Vorrichtung vereinfacht werden.

In einer besonders bevorzugten Ausführungsform wird die Spaltabbildung mittels eines Projektionsverfahrens, vorzugsweise mittels DLP erzeugt. Damit kann auf mechanische Teile verzichtet werden, womit die Vorrichtung wiederum kostengünstiger ausgebildet werden kann.

In Varianten kann auf den Filter respektive auf die motorische Betätigbarkeit auch verzichtet werden. Eine Spaltbeleuchtung kann mittels einer herkömmlichen, mechanischen Blende erzeugt werden.

Die Vorrichtung kann insbesondere weiter eine Steuerungseinheit umfassen, womit die Spaltform und/oder die Filterwahl automatisch gewählt respektive anhand der gemessenen Daten automatisch angepasst und optimiert werden können.

Vorzugsweise sind die Spaltbeleuchtung und die Sensoren derart synchronisierbar, dass die Spaltbeleuchtung gleichzeitig mit den Sensoren, insbesondere während der Belichtungszeit, aktiviert wird. Insbesondere bei der Betrachtung eines Auges können damit unerwünschte Reaktionen und Reflexe des Auges, wie zum Beispiel das Öffnen der Pupille etc., während der Bildaufnahme vermieden werden. Damit werden im Verfahren vorzugsweise die Beleuchtung und die Sensoren gleichzeitig aktiviert. Unter dem Begriff "gleichzeitig" sind vorliegend kleine zeitliche Abweichungen, welche die Bildaufnahme nicht beeinträchtigen, zulässig. So kann zum Beispiel die Beleuchtung im Millisekunden Bereich vor der Aktivierung der Sensoren aktiviert werden, um zu vermeiden, dass aufgrund der gegebenen Fehlertoleranzen die Sensoren vor der Beleuchtung aktiviert werden. Ebenso kann die Beleuchtung zeitlich geringfügig nach dem Deaktivieren der Bildsensoren deaktiviert werden. Damit wird sichergestellt, dass die Beleuchtung während der Belichtungszeit der Sensoren aktiv ist.

In Varianten kann auf die Synchronisierbarkeit der Beleuchtung und der Sensoren auch verzichtet werden.

In einem Verfahren zum Ermitteln von Bilddaten werden die folgenden Schritte durchgeführt:
- Aufnehmen von Bilddaten eines Objektes durch den ersten Sensor und den zweiten Sensor, insbesondere bei unterschiedlicher Perspektive;
- Verarbeiten der Bilddaten des ersten Sensors und des zweiten Sensors, insbesondere zu einem einzigen Bild und/oder zu einer Bildsequenz respektive zu einem einzigen Bildausschnitt und/oder zu einer Bildausschnittsequenz.

Durch die Verarbeitung der Bilddaten des ersten und des zweiten Sensors zu einem einzigen Bild kann zum Beispiel die Bildqualität verbessert werden. So können zum Beispiel die Bilddaten zweier Bildsensoren zu einem Bild mit einer gegenüber den Einzelbildern verbesserter Bildqualität verrechnet werden. Die beiden Sensoren und/oder die Objektive können sich jedoch auch in einer Eigenschaft unterscheiden, um bei dem verrechneten Bild von den Vorteilen der unterschiedlichen Eigenschaften, welche sich unter Umständen bei einem einzelnen Bildsensor ausschliessen können, profitiert werden kann.

Vorzugsweise werden die Bilddaten des Objekts zeitgleich durch den ersten Sensor und den zweiten Sensor aufgenommen. Insbesondere bei sich bewegenden Objekten, wie zum Beispiel bei einem Auge, können damit Bilddaten erreicht werden, welche das Objekt in denselben Zustand und in derselben Ausrichtung zeigen. Damit können die Bilddaten der einzelnen Sensoren in einfacher Weise zu einem einzigen Bild verrechnet werden, da Bewegungsartefakte nicht oder kaum berücksichtigt werden müssen.

In Varianten können mit den Sensoren die Bilddaten auch zeitlich hintereinander aufgenommen werden.

Vorzugsweise unterscheiden sich die Aufnahmebedingungen des ersten Sensors und des zweiten Sensors, insbesondere in einem der folgenden Merkmale:
a. Belichtungszeit;
b. Blendeneinstellung;
c. Filtereinstellung;
d. Aufnahmespektrum.

Durch eine Variation der Aufnahmeeigenschaften des ersten Sensors und des zweiten Sensor können Bilddaten erhalten werden, welche in unterschiedlichen Bereichen höhere Qualitäten aufweisen. Damit können insbesondere Einstellungen vorgenommen werden, welche sich bei einem einzigen Sensor widersprechen würden.

Zum Beispiel kann für den ersten Sensor eine lange Belichtungszeit gewählt werden, um eine hohe Bildqualität zu erreichen, während mit dem zweiten Sensor eine kurze Belichtungszeit gewählt wird, um möglichst keine Bewegungsartefakte und damit ein scharfes Bild zu erreichen. Mit einer Kombination der Bilddaten des ersten und des zweiten Sensors können nun die Vorteile der beiden Einstellungen zu einem einzigen Bild kombiniert werden.

In einem weiteren Beispiel kann für den ersten Sensor eine grosse Blendenöffnung gewählt werden, um einen grossen Lichteinfall auf den ersten Sensor zu erhalten, während für den zweiten Sensor eine kleinere Blendenöffnung gewählt wird, um eine möglichst grosse Tiefenschärfe zu erreichen. Wiederum können durch die Verarbeitung der Bilddaten die Vorteile der jeweiligen Einstellungen in einem einzigen Bild kombiniert werden.

Weiter können bei den beiden Sensoren verschiedene Filter, zum Beispiel Farbfilter, eingesetzt werden, wobei bei einer Verarbeitung der Bilddaten zu einem einzigen Bild die Vorteile der einzelnen Filter kombiniert werden können. Durch eine geeignete Wahl von Farbfiltern können zum Beispiel gezielt Kontraste verändert werden.

Durch die Wahl von unterschiedlichen Aufnahmespektren für die beiden Bildsensoren können wiederum unterschiedliche Kontraste hervorgeholt werden. Die Bilddaten können wiederum zu einem einzigen Bild verarbeitet werden, in welchem die Vorteile der einzelnen Aufnahmespektren zu einem Bild kombiniert werden können.

Dem Fachmann sind weitere Aufnahmebedingungen bekannt, welche beim ersten Sensor und beim zweiten Sensor unterschiedlich gewählt werden können. Die Vorteile ergeben sich insbesondere bei einer im Wesentlichen zeitgleichen Aufnahme desselben Bildausschnittes. Dem Fachmann ist aber klar, dass die Aufnahmen nicht zwingend zeitgleich erstellt werden müssen, um die Bilddaten zu einem einzigen Bild kombinieren zu können. Auch müssen die Bilddaten nicht zwingend zu einem einzigen Bild kombiniert werden, sondern können alternativ oder zusätzlich auch eigenständig verwendet respektive verarbeitet werden. Weiter müssen nicht zwingend Einzelbilder verarbeitet werden, es können auch Bildsequenzen der beiden Sensoren zu einer einzigen Bildsequenz oder auch zu einem einzigen Bild verarbeitet werden. Mit den Stereobildern können 3D-Effekte über Motion-Parallax erreicht werden, wobei aus zwei oder mehreren 2D-Bildern und eventueller Interpolation eine 2D-Bildsequenz erstellt wird, die dem Betrachter einen 3D-Eindruck vermittelt. In Varianten können anstelle von Stereobildern auch Stereobildsequenzen verwendet werden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Darstellung einer Draufsicht einer ersten Ausführungsform einer Vorrichtung zur Untersuchung eines Auges; und
- Fig. 2: eine schematische Draufsicht auf einen Optikteil mit drei Objektiven.

Grundsätzlich sind in den Figuren gleiche Teile mit gleichen Bezugszeichen versehen.

### Wege zur Ausführung der Erfindung

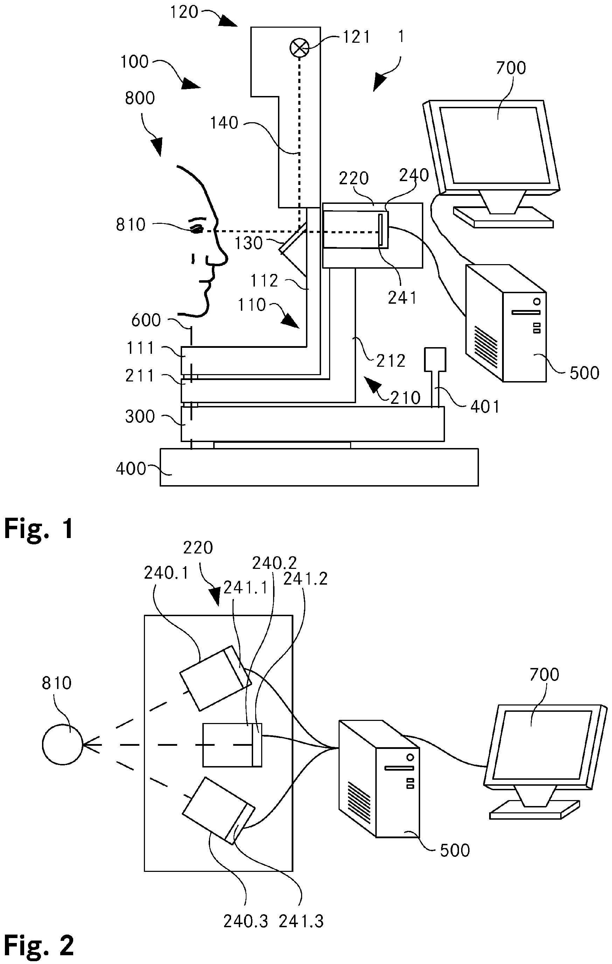

Die Figur 1 zeigt eine Vorrichtung 1 zur Untersuchung eines Auges, ausgebildet als Spaltlampe 1. Die Spaltlampe 1 umfasst eine Beleuchtungseinheit 100, einen Optikteil 220 und zwei Objektive 240 welche auf einem Kreuzschlitten 300 montiert sind. Der Kreuzschlitten 300 selbst ist auf einer Grundplatte 400 montiert, welche als Tischplatte ausgebildet sein kann, und kann in X-, Y- und Z-Richtung verfahren werden. Der Schlitten 300 ist über ein Bedienelement 401, welches an demselben angeordnet ist, steuerbar. Die Beleuchtungseinheit 100 und die beiden Objektive 240 sind damit über den Schlitten 300 verfahrbar sowie über eine gemeinsame Drehachse 600 unabhängig voneinander verschwenkbar angeordnet.

Die Beleuchtungseinheit 100 für die Spaltbeleuchtung umfasst ein L-förmiges Element 110 mit einem horizontalen Abschnitt 111 und einem vertikalen Abschnitt 112. In einem distalen Bereich des horizontalen Abschnittes 111 umfasst das L-förmige Element 110 eine vertikal orientierte Drehachse 600. Oben auf dem vertikalen Abschnitt ist eine Beleuchtungsvorrichtung 120, umfassend eine Lichtquelle 121, angeordnet. Die Beleuchtungseinheit 100 ist so beschaffen, dass ein definierter Lichtstreifen erzeugbar ist, welcher auf ein Auge 810 projiziert werden kann. An der Innenseite des L-förmigen Elements 110, am vertikalen Abschnitt 112 befindet sich ein Spiegel 130, welcher sich vom vertikalen Abschnitt 112 aus in einem Winkel von 45° neigt. Ein durch die Lichtquelle 121 erzeugter Lichtstrahl 140 wird senkrecht nach unten auf den in einem 45°-Winkel angeordneten Spiegel 130 geleitet und von diesem zum Auge 810 des Patienten 800 geleitet. Dem Fachmann ist klar, dass die Beleuchtungseinheit auch vertikal unterhalb des Spiegels angeordnet sein kann, wobei der Spiegel um einen Winkel von 90° verschwenkt würde. Anstelle eines Spiegels können auch andere optische Elemente, mit analoger Funktion verwendet werden. Die Beleuchtungseinheit 100 kann motorisch oder von Hand um die Achse 600 verschwenkbar sein. Die Beleuchtungseinheit 100 ist in einer bevorzugten Ausführungsform als DLP-Projektionsvorrichtung ausgebildet und wird rein elektronisch gesteuert - dem Fachmann ist jedoch klar, dass auch herkömmliche mechanische Vorrichtungen zur Spalterzeugung vorgesehen sein können.

Die Bilddaten werden von den beiden Bildsensoren 241 der Objektive 240 in der vorliegenden Ausführungsform an einen Computer 500 gesandt. Dies kann entweder über Kabel oder in bekannten Weisen auch kabellos erfolgen. Der Computer 500 ist vorliegend als separates Gerät dargestellt. Der Computer 500 kann aber auch in Form einer Recheneinheit Bestandteil der Vorrichtung sein. Ebenso ist vorliegend ein Bildschirm 700 mit dem Computer 500 verbunden. Auch dieser kann Bestandteil der Vorrichtung sein. Insbesondere können statt des Bildschirms 700 auch andere dem Fachmann bekannte Mittel zur Betrachtung von digitalen Bilddaten vorgesehen sein.

Die vom Auge 810 zurückkommenden Lichtstrahlen, gelangen jeweils in einen Optikteil 220, welches vorliegend zwei Objektive 240 umfasst. Ein Objektiv 240 umfasst jeweils einen Bildsensor 241, welcher einteilig oder mehrteilig ausgebildet sein kann. Bei den beiden Objektiven 240 handelt es sich vorliegend um Fixobjektive, welche eine fixe Vergrösserung aufweisen und zudem fix im Strahlengang angeordnet sind. Bei den Sensoren handelt es sich vorliegend um jeweils einen Farbsensor. Weiter ist vorliegend bei den Objektiven 240 eine Fokusebene einstellbar, insbesondere ist die Fokusebene umschaltbar.

Die Figur 2 zeigt eine schematische Darstellung einer zweiten Ausführungsform eines Optikteils 220, umfassend drei Objektive 240.1, 240.2 und 240.3. Die drei Objektive 240.1, 240.2 und 240.3 sind jeweils auf das Auge 810 gerichtet, wobei sich die Fokusebenen der drei Objektive 240.1, 240.2 und 240.3 in einer gemeinsamen Achse schneiden. Die Objektive 240.1 und 240.3 sind aussen angeordnet und schliessen das Objektiv 204.2 ein, welches zentral auf das Auge 810 gerichtet ist. Mit den Objektiven 240.1 und 240.3 können stereoskopische Abbildungen generiert werden. Die Sensoren 241.1, 241.2 und 241.3 der Objektive 240.1, 240.2 und 240.3 sind mit einem Computer 500 verbunden. Die Sensoren 241.1, 241.2 und 241.3 sind mittels einer Recheneinheit respektive des Computers 500 derart ansteuerbar, dass Bilder im Wesentlichen zeitgleich aufgenommen werden können. Zudem wird mit der Recheneinheit gleichzeitig die Beleuchtungseinheit 100 angesteuert, so dass die Sensoren und die Beleuchtungseinheit 100 zeitgleich aktiviert werden können. Damit können Störungen der Aufnahmen durch Reflexe mittels Bildbearbeitung weitgehend ausgeblendet werden.

In einer ersten Variante sind die Objektive 240.1 und 240.3 identisch ausgebildet und umfassen einen Farbsensor 241.1 respektive 241.3, während das Objektiv 240.2 einen Schwarzweiss-Sensor 241.2 umfasst und mit einer grösseren Vergrösserung ausgestattet ist. Damit kann mit den beiden Objektiven 240.1 und 240.2 ein Übersichtsbild erhalten werden. Mit dem Sensor 241.2 des Objektivs 240.2 können Details innerhalb des Übersichtbildes mit grosser Auflösung wiedergeben werden. Das Bildmaterial des Objektivs 240.2 kann anhand der Daten der beiden Objektive 240.1 und 240.3 koloriert werden.

In einer zweiten Variante sind die Objektive 240.1 und 240.3 mit grosser Vergrösserung und mit Schwarzweiss-Sensoren 241.1 respektive 241.3 ausgestattet, während das Objektiv 240.2 mit einem Farbsensor 241.2 ausgestattet ist und eine geringere Vergrösserung aufweist. Damit ist in dieser zweiten Variante das Objektiv 240.2 zur Erstellung eines Übersichtsbildes ausgebildet, während mit den beiden Objektiven 240.1 und 240.3 detaillierte stereoskopische Aufnahmen des Auges in hoher Auflösung gemacht werden können.

In einer dritten Variante sind sämtliche Sensoren 241.1, 241.2 und 241.3 der drei Objektive 240.1, 240.2 und 240.3 als Farbsensoren und in einer vierten Variante als Schwarzweiss-Sensoren ausgebildet, wobei jeweils das Objektiv 240.2 eine geringere Vergrösserung aufweist, als die Objektive 240.1 und 240.3.

In einer fünften Variante umfasst das Objektiv 240.2 einen IR-Sensor 241.2, während die Objektive 240.1 und 240.3 jeweils einen Farbsensor 241.1 respektive 241.3 umfassen. In einer sechsten Variante umfasst das Objektiv 240.2 einen IR-Sensor 241.2, während die Objektive 240.1 und 240.3 jeweils einen Schwarzweiss-Sensor 241.1 respektive 241.3 umfassen.

In einer siebten Variante sind die einzelnen Objektive fix zueinander angeordnet, während in einer achten Variante mindestens eines der Objektive jeweils um eine Achse verschwenkbar ist. Vorzugsweise sind zumindest die Objektive 240.1 und 240.3 um parallele Achsen in der vertikalen Ebene verschwenkbar, während das Objektiv 240.2 fix angeordnet ist, insbesondere in einer Ausführungsform, in welcher das Objektiv 240.2 zum Erfassen eines Übersichtbildes ausgebildet ist. Dem Fachmann sind beliebig weitere Varianten bekannt.

Dem Fachmann ist des Weiteren klar, dass statt der genau zwei oder drei Objektive auch mehr als drei Objektive vorgesehen sein können. Die Objektive können auch derart angeordnet sein, dass deren optischen Achsen parallel oder nahezu parallel verlaufen, um einen grösseren Bildbereich abdecken zu können.

Zusammenfassend ist festzustellen, dass erfindungsgemäss eine Vorrichtung zur Untersuchung eines Auges bereitgestellt wird, welche sich durch einerseits besonders präzise und robuste Aufnahmen und anderseits durch einen einfachen und kostengünstigen Aufbau auszeichnet.

## Patentansprüche

1. Vorrichtung (1) zur Untersuchung eines Auges, insbesondere eine Spaltlampe, umfassend eine Bildaufnahmeeinheit (220) mit mindestens einem ersten Sensor (241.1) in einem ersten Strahlengang und einem zweiten Sensor (241.2) in einem zweiten Strahlengang, **dadurch gekennzeichnet, dass** sie im ersten Strahlengang weiter ein erstes Objektiv mit einer ersten optischen Achse umfasst, welches eine fixe Vergrösserung aufweist und fix im ersten Strahlengang angeordnet ist.

2. Vorrichtung (1) nach Anspruch 1, **dadurch gekennzeichnet, dass** das erste Objektiv den ersten Sensor (241.1) umfasst und die Vorrichtung (1) weiter ein zweites Objektiv mit einer zweiten optischen Achse umfasst, wobei das zweite Objektiv den zweiten Sensor (241.2) umfasst sowie insbesondere eine fixe Vergrösserung aufweist und fix im zweiten Strahlengang angeordnet ist.

3. Vorrichtung (1) nach Anspruch 1 oder 2, **dadurch gekennzeichnet, dass** sich der erste Sensor (241.1) und der zweite Sensor (241.2) in einem Aufnahmespektrum unterscheiden.

4. Vorrichtung (1) nach Anspruch 3, **dadurch gekennzeichnet, dass** der erste Sensor (241.1) als Farbsensor ausgebildet ist und der zweite Sensor (241.2) als Schwarzweiss-Sensor oder als IR-Sensor ausgebildet ist.

5. Vorrichtung (1) nach einem der Ansprüche 1 bis 4, **dadurch gekennzeichnet, dass** sie mindestens einen dritten Sensor (241.3) in einem dritten Strahlengang aufweist, welcher insbesondere durch ein fix im dritten Strahlengang angeordnetes drittes Objektiv mit vorzugsweise fixer Vergrösserung umfasst ist, wobei insbesondere der zweite Sensor (241.3) als Farbsensor und der dritte Sensor (241.3) als IR-Sensor ausgebildet sind.

6. Vorrichtung (1) nach einem der Ansprüche 1 bis 5, **dadurch gekennzeichnet, dass** ein erster Vergrösserungsfaktor des ersten Objektivs kleiner ist, als ein zweiter Vergrösserungsfaktor des zweiten Objektivs.

7. Vorrichtung (1) nach einem der Ansprüche 1 bis 6, **dadurch gekennzeichnet, dass** sich ein Bildaufnahmebereich in einer Fokalebene des ersten Strahlengangs mit einem Bildaufnahmebereich in einer Fokalebene des zweiten Strahlengangs zumindest teilweise überlappt.

8. Vorrichtung (1) nach Anspruch 7, **dadurch gekennzeichnet, dass** der Bildaufnahmebereich in der Fokalebene des zweiten Strahlengangs innerhalb des Bildaufnahmebereichs in der Fokalebene des ersten Strahlengangs liegt.

9. Vorrichtung (1) nach Anspruch 2, **dadurch gekennzeichnet, dass** ein Winkel zwischen der ersten optischen Achse und der zweiten optischen Achse einstellbar ist.

10. Vorrichtung (1) nach Anspruch 9, **dadurch gekennzeichnet, dass** die erste optische Achse und die zweite optische Achse parallel einstellbar sind.

11. Vorrichtung (1) nach einem der Ansprüche 1 bis 10, **dadurch gekennzeichnet, dass** in der ersten optischen Achse und in der zweiten optischen Achse eine Blende und/oder ein optischer Filter angeordnet sind, welche insbesondere motorisch betätigbar sind.

12. Verfahren zum Ermitteln von Bilddaten mit einer Vorrichtung (1) nach einem der Ansprüche 1 bis 11, **gekennzeichnet durch** die folgenden Schritte:
a. Aufnehmen von Bilddaten eines Objektes durch den ersten Sensor (241.1) und den zweiten Sensor (241.2), insbesondere bei unterschiedlicher Perspektive;
b. Verarbeiten der Bilddaten des ersten Sensors (241.1) und des zweiten Sensors (241.2), insbesondere zu einem einzigen Bild und/oder zu einer Bildsequenz respektive zu einem einzigen Bildausschnitt und/oder zu einer Bildausschnittsequenz.

13. Verfahren nach Anspruch 12, **dadurch gekennzeichnet, dass** die Bilddaten des Objekts zeitgleich durch den ersten Sensor (241.1) und den zweiten Sensor (241.2) aufgenommen werden.

14. Verfahren nach Anspruch 12 oder 13, **dadurch gekennzeichnet, dass** Bilddaten eines als Schwarzweiss-Sensor ausgebildeten ersten Sensors (241.1) mit einer ersten Auflösung mit Bilddaten eines als Farb-Sensor ausgebildeten zweiten Sensors (241.2) zu einem einzigen kolorierten Bild und/oder zu einer kolorierten Bildsequenz verrechnet werden, wobei der zweite Sensor (241.2) eine Auflösung aufweist, welche insbesondere geringer ist als die Auflösung des ersten Sensors (241.1).

15. Verfahren nach einem der Ansprüche 12 bis 14, **dadurch gekennzeichnet, dass** sich die Aufnahmebedingungen des ersten Sensors (241.1) und des zweiten Sensors (241.2) unterscheiden, insbesondere in einem der folgenden Merkmale:
a. Belichtungszeit;
b. Blendeneinstellung;
c. Filtereinstellung;
d. Aufnahmespektrum.
